# EUROPEAN PATENT APPLICATION

(11) **EP 4 105 937 A1**
(43) Date of publication of application: **21.12.2022**
(21) Application number: 21472003.9
(22) Date of filing: 16.06.2021
(51) Int. Cl.: G16H 10/60, G16H 10/65

(54) **MODULE SCANNER DEVICE FOR VERIFICATION OF COVID 19 STATUS**

(71) Applicant: TEOTONIO Ltd, Sofia (BG)
(72) Inventor: Yankov Dzhoganov, Aleksandar, Sofia (BG); Boikova Nikolova, Iskra, Sofia (BG); Strahilov Yosifov, Ivaylo, Sofia (BG); Kolev Petkov, Petar, 1309 Sofia (BG)
(74) Representative: Ivanov, Ivan Nikolov

(57) **Abstract**

The present invention relates to a module scanner device for fast, secure and convenient verification of COVID-19 status (vaccinated, ill, with a negative PCR test, etc.) of persons travelling in the country or abroad or visiting public buildings or events, or private or public accommodation or feeding, or in general, in any place where clarity is required as to the status of the person and his admission (or non-admission) to certain activities without the need for additional testing or quarantine.

Scanner device for verification of Covid 19 status, according to the claimed invention is composed of scanner module (1) provided on one hand with bidirectionally connected to a control programming interface (2) that is bidirectionally connected to a database server (3) and an application server (4), and on another hand the scanner module (1) is also bidirectionally connected to a module integration hub (5), wherein the module integration hub (5) and the server with the application for scanners' management (3) are connected via the Internet connection with the API's of third parties (7), provided with functionalities to extraction data from bidirectionally secured via encrypted internet protocol or a private VPN third parties' databases (9) and wherein the module scanner (1) includes a control module (17) responsible also for the management of the communication module that is connected to the Internet, and is functionally bidirectionally connected via a user interface (18) with a touch-screen (19).wherein a document (20), a QR code (21), camera (23) and a biometric scanners (22) are connected to the control module (17).

## Description

### Field of the invention

The present invention relates to a device for fast, secure, and convenient verification of COVID-19 status (vaccinated, ill, with a negative PCR test, etc.) of persons travelling in the country or abroad or visiting public buildings or events, or private or public accommodation or feeding, or in general, in any place where clarity is required as to the status of the person and his admission (or non-admission) to certain activities without the need for additional testing or quarantine.

### Background of the Invention

The danger of the COVID-19 pandemic has not passed, and as announced by the WHO, this will probably not be the last pandemic that awaits us in the next few years.

There is an increasing need for reliable and clear information to serve both national and European health authorities and all those involved in the process. The possibility for easy, fast, and secure identification of the health status of individuals is becoming increasingly important for the normalization of the economy, tourism, and the free movement of people, so that this does not slow down the processes of crossing borders or visiting public places. This will require a unified method that gives equal rights to everyone, regardless of whether they have a smart device or a paper document.

Finally, is the extreme sensitivity of people's personal data - who and under what circumstances can see personal data such as names, ID number, number of identity documents, their validity, etc. while avoiding attempts at falsification (e.g., issuing false AKP tests), attempts to display a unknown document (from different country or authority) or QR code. If the person's data is completely anonymized and shows only some status (for example - vaccinated, active AKP test, with antibodies) then this QR code could be copied and used to identify other persons who do not have the appropriate status.

In the case of anonymised data (YES or NOT only) there is a strong contradiction between public health care and the need for significant protection of personal data.

In view of the continued COVID-19 pandemic and it's impact in Europe and the world, both in terms of morbidity and mortality and in terms of severe social and economic consequences a key role in improving the public health response in this situation is expected to be its future digitalisation, a task that is a priority for the WHO Regional Office for Europe for the period 2020-2025. The digitalisation of public health allows the introduction of innovative data collection methods and the interconnection between institutions, for data visualization and analysis.

Non-pharmaceutical restrictive measures will continue to be in force and will vary in time and place for all countries, in Europe and world-wide and within countries. In this situation, the individual immune status (immunization certificate/passport) determined by laboratory data for testing against SARS CoV-2 and the presence of antibodies or immunization against the disease can be used to facilitate cross-border travel but also domestic travel (within countries).

In this regard, the EC announced that a common database of EU countries is being created to record the status of individuals (vaccinated, with an active laboratory test, sick, etc.) to avoid attempts of fraud (e.g. fake test, fake vaccination documents, etc.)

Currently, the identification of the health status of individuals is done through:
- A paper document with a QR code issued by an institution authorized and licensed by the respective national authority (hospital, GP, regional health inspectorate, licensed testing laboratories, etc.) This paper document reflects the current situation as of a certain date and is recorded in specialised databases by each institution and country, which is authorized to do so with deliberate registration in the respective country. This can be a document for completed vaccination, a document for past illness or a test with a certain period of validity.
- A QR code that can be downloaded to a smart device. This code is generated from databases and downloaded by the person concerned or a photo of the QR code is taken from the paper document. When downloading - cuts the current state, but when taking a photo gives information only about some momentary state back in time.
- Through a platform for checking and visualizing a health condition based on vaccinations tests. Test results include information on the absence or presence of an infectious disease in the tested person, time and date of sampling, and location. The platform issues a certificate, which must be controlled by external specialists, who have the right to validate that the identity of the person reported as tested; that the materials used for the test have been approved by the respective government authorities.
- The data from the platform is encrypted in a separate database and can be read on request it and the result can be visualized.

U.S. Patent No. 10,963,216, of Feb. 16, 2021, discloses a health status platform that includes a receiving component that receives a test result, a test of a biological sample collected from a patient. The test result includes an indication of the presence of an infectious disease in the patient, as well as the identification and examination of the patient. The platform includes a certificate component that issues a certificate of origin for the biological sample; and a data aggregation component that collaborates with a site access manager that controls site access. The data aggregation component implements a distributed book system that stores encrypted patient test results and patient identification and verification, and an end-to-end encryption system that receives an encrypted site access request from the site access manager, decrypts the access request, determines whether the access request is valid and, if valid, provides an encrypted certificate of origin to the site access manager.

Disadvantage of QR code on a paper document:
- There are thousands of laboratories in the EU that perform PCR testing, each of which is licensed in the respective country. When crossing the border, the relevant verifier must check in a special list whether the institution that issued the document is licensed and has the rights and permission to issue such a document. It is no less important that the document can be easily falsified by making a similar one or that the institution that made it has lost its business license on time.
- The document may be damaged or lost.
- Many personal data - names, personal ID, address, and others that are essential for identification, but are not always acceptable for access by any verifier.
- It may be in a language that is different from the language of the examiner, and this may lead to certain misunderstandings.
- Needs a reliable reader, if machine-readable, to protect personal data.

Disadvantage of smart media with QR code:
- Not everyone has a smart device.
- The battery may be depleted at the wrong time and the smart device may not be able to display the code.
- Need for roaming connection if the person being checked is abroad and the QR code is downloaded each time on request.
- Need for deliberate registration to identify the person and which must be reliably protected to prevent tampering.
- It can be manipulated as no one has control over the device.
- Needs a reliable reader, if machine-readable, to protect personal data.

Disadvantage of test and visualization platform:
- In each country, points or deliveries of machines must be organized to be certified by the respective country.
- There must be deliberate, complex, and secure logistics of delivery of consumables to the devices that perform the test.
- A secure database must be created and maintained, agreed upon between many and different countries.
- This could create a problem with the protection of personal data.
- If a consumable is not appropriate, it may result in false positive or false negative test.
- A database of person who have been vaccinated or have had an infectious disease is not included and according to the WHO and the health authorities, there is a certain period in which these person can be considered not to transmit the infection.

### SUMMARY OF THE INVENTION

The claimed invention subject to this patent application relates to a device that is capable of easy, fast, and secure verification of the status of persons to establish the absence or presence of an infectious disease (including for exampleCOVID-19), that is previously noted in specialized medical databases of the respective country or countries.

The device, according to this patent application, can be installed at any point where identification of a person and his health status is required. It reduces the time for processing documents, minimizes the risk of fraud or presentation of a false document (for vaccination, for a test, for a past illness, for the presence of antibodies), it can provide information (non-anonymised or anonymised data).

In the EU the freedom of movement is a basic right. However, in the last 18 months, the COVID-19 pandemic has disrupted such right. Even now when the vaccination process is going on in all countries and there is already an EC regulation for the so-called "Green certificates" there are still have a few unresolved issues, for example:
- If someone loses the paper document and is outside the borders of their country, how will they obtain a duplicate and certify their health status?
- If someone does not own a smart device or does not have access to roaming, how will they show the QR code generated on the device?
- Who and with what devices will check the availability and authenticity of the certificates, what personal data will be accessed and is there no danger if this device is not certified and verified, to be attacked and very sensitive personal information to leak?

The device, subject to this invention, and presented in this patent application, solves all these issues and can be used in various places such as, including, but not limited to state borders crossings, admission to an airflight, admission to government, municipal or private institutions, separation of flows of "Green" and "Red" persons at public events or on visits to the public or similar buildings.

The use of the device according to the present patent application could be fully automate, without the need for human intervention, for example for verification of identity or test results. This will be undertaken by integrating with already built software for access mode in public places, buildings, or events.

The object of the present invention is to provide a device for enabling quick, easy, secure and safe identification of persons and their health status in real-time. The device, according to the present European patent application, should limit the possibilities for manipulation or forgery, be reliably protected from personal data breach and be used practically anywhere, since the device has different size options and different status visualization options.

Of the existing methods for identification and verification of health status listed above, the device subject to this European patent application solves the existing practical problems as follows:

### The device

1. Will identify unambiguously the person in front of the device by an identity document (e.g., id card, passport, driver's license) by checking the authenticity of the document and an additional method of identifying the person in front of the device such as facial recognition, comparing the identity document with a database of the country of origin and confirming the document is genuine and valid, comparing biometric data with a database of the country of origin or with iris face recognition by connecting to a database of the country of origin.
2. Will visualise only the necessary and allowed for access to the relevant verifier information and which, in some cases, can be anonymized. That is - the device validates that the person in front of it is the holder of the identity document and gives information on the screen only about the status - for example:
   - green - a second dose of the corresponding vaccine has been given and the required number of days have passed to assume that the person is not contagious.
   - blue - the person has had an infectious disease, the required number of days has elapsed after being diagnosed as healthy, and the required number of days has not yet elapsed to require a vaccine or test.
   - yellow - the person has an active test, which is within the period set by the health authorities of the country.
   - red - no information is available for any of the above statuses.
   The device can only validate the person's identity and read a QR code from a paper or smart device.
3. Does not require complex logistics as in the known patent document referred to in the prior art part above.
4. Does not need a single database, but can be connected to various ones and, depending on the legislation and regulations for personal data protection, to visualize only the necessary information by visualization on a screen or by a light signal.
5. Works with over 70 languages and information can be displayed in the language of the examiner.
6. Stops all possibilities of forgery or presentation of documents by unlicensed institutions, without the verifier having to do so.
7. Does not contain any personal data of any of the inspected persons and there is no danger of misuse.
8. Transmits data with encrypted connection via mobile VPN.
9. Would give reliable and accurate information about the status of the person even if there is no paper document or QR code on the smart device.
10. Software and hardware can be easily upgraded to expand its capabilities by adding new features.

### Brief description of the attached figures

This invention is illustrated in the accompanying figures, where:
- Figure 1 is a schematic diagram of the system.
- Figures 2 and 3 are block diagrams of the process of using the disclosed device, according to the invention.
- Figure 4 is a schematic diagram of the hardware of the device, according to the invention.

### Preferred embodiment of the invention

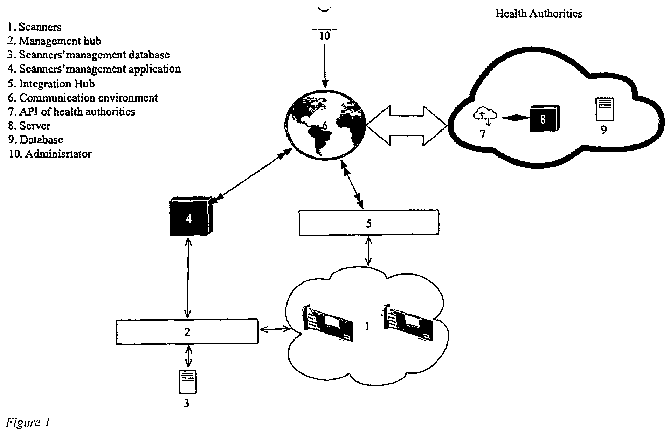

The module of scanners shown in Figure 1 are bidirectionally connected to a control programming interface (2) that is bidirectionally connected to a database server (3) and an application server (4).

Scanners are also bidirectionally connected to an integration hub (5). The integration hub (5) and the server with the application for scanners' management are connected via the Internet with the APIs of third parties (7). These APIs (7) provide functionalities to extract data from third parties' databases (9). The connection is secured via encrypted internet protocol. In the case of mobile data, a private VPN over the public network is established.

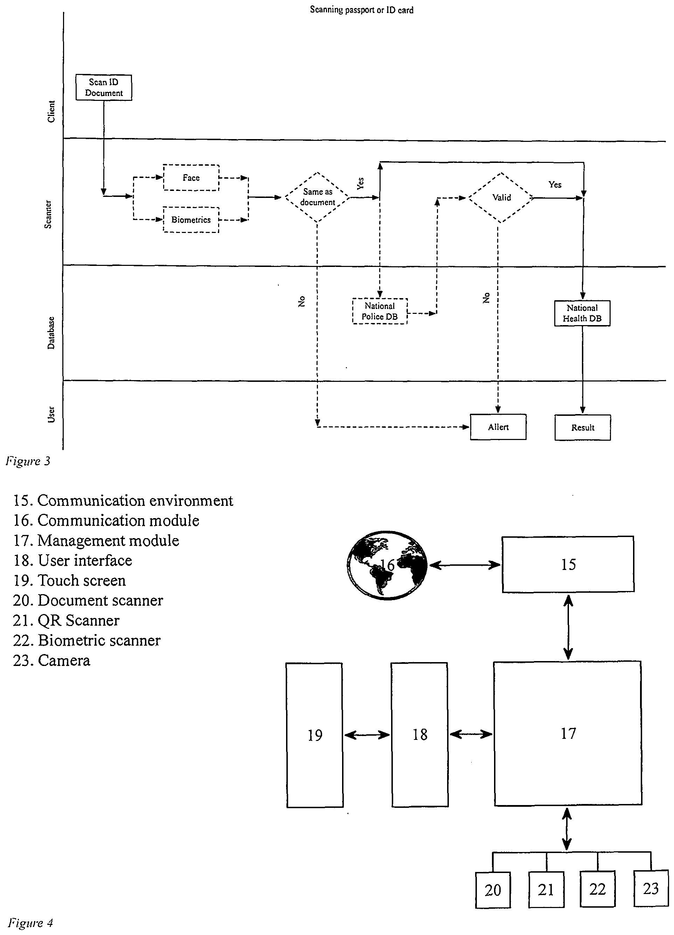

Each device, shown in Figure 4, includes a control module (17), which is functionally bidirectionally connected via a user interface (18) with a touchscreen (19).

The control module (17) is also responsible for the management of the communication module that connects to the Internet. The communication module includes the following adapters:
- Wi-Fi
- 3G/4G
- Ethernet

A document (20), a QR code (21) and biometric scanners (22) are connected to the control module (17).

The document scanner recognises over 8,000 types of documents (ID cards, passports, driving licenses, etc.) from over 240 countries and supports over 70 languages. It also supports/ recognises Latin, Cyrillic, Hebrew, Greek, and other alphabets. The scanner includes a QR code reader that is only capable to read QR code from paper. Since it cannot recognise QR code from smart devices, an additional QR code reader (22) is included.

A camera (23) for face recognition is connected to the control module (17).

The usage of the device, according to the invention provides two main scenarios that are possible with the proposed device configuration:

### Scenario A: health certificate scanning

In this scenario, the health certificate QR code is scanned. The certificate number extracted from the code was sent to the national health database. The device received the response from the database and visualised only permitted information on the screen. The following scenarios are possible, but not exhaustive:
- All available data were presented
- Only colour lights signal depending on the status was shown
- A door in a public place is opened, etc.

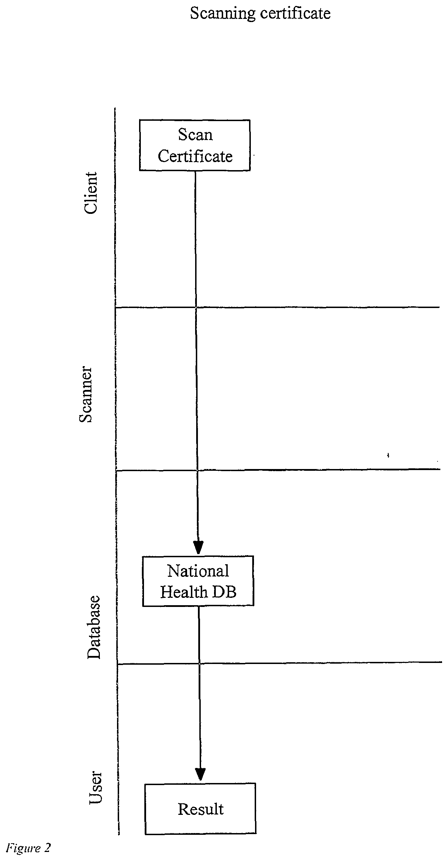

This scenario is illustrated in Figure 2.

### Scenario B: personal identification document scanning

This could be a passport or national identification card or driving license. The process is shown in Figure 3. Depending on the legislation and technical capabilities the process could be more complex or simpler. The optional steps are indicated with dashed lines and the mandatory are with normal lines.

The simple scenario performs the following steps:
1. Scanning of the document
2. Extracting identification data
3. Connecting to the national health database.
4. Extracting the data
5. Comparing the data
6. Presenting the result.

The following scenarios are possible, but not exhaustive:
- All available data were presented
- Only colour lights signal depending on the status was shown

A door in a public place is opened, etc.
- This scenario is illustrated in Figure 2.

The following steps could be taken in addition to identify the person more securely:
1. Compare the picture of the document and the picture taken by the camera of the device.
2. Perform a biometric scan and compare it with the data in the scanned document (if it stores biometric data) or with the national ID database.
3. Check with the national ID database if the presented document is valid.

The presented process has the following characteristics:
- The process is fully automated and works around the clock.
- Does not require pre-registration.
- It can show status even if a paper document is lost or if it is impossible to display a QR code on a smart device for some reason.
- If more information is displayed, it is set in the language of the examiner.
- Centralized management and easy modification.
- If necessary, it can be upgraded easily and remotely.
- Do not keep any personal information.
- Different modifications of the device can be placed in different places so that personal data can be displayed only where necessary or where there is permission (e.g., border crossings).
- The device cannot be manipulated and can provide important and necessary statistics to authorized persons without containing any personal data, for example: through the specific device, there are 100 status checks, of which 50 vaccinated, 30 with a negative test and 20 for which has no data.

### The advantages of the invention:

In connection with the adopted decision of the EC for a single digital certificate, the device according to the invention allows for fast, reliable, and secure verification of the status of the inspected person, so that on the one hand to avoid possible manipulations and on the other - to protect personal data.

The device according to the invention is multilingual and can provide information in the language of the examiner, so that there are no misunderstandings due to the language.

Fast and reliable connection, protected by encryption.

## Claims

1. Scanner device for verification of COVID-19 status, **characterized in that** it is composed of a scanner module (1) provided on one hand with bidirectionally connected to a control programming interface (2) that is bidirectionally connected to a database server (3) and an application server (4),

2. Scanner device for verification of Covid 19 status, **characterised in that** it is composed of a scanner module (1) bidirectionally connected to a module integration hub (5), wherein the module integration hub (5) and the server with the application for scanners' management (3) are connected via the Internet connection with the API's of third parties (7), provided with functionalities to extraction data from bidirectionally secured via encrypted internet protocol or a private VPN third parties' databases (9) and wherein the module scanner (1) includes a control module (17) responsible also for the management of the communication module that is connected to the Internet, and is functionally bidirectionally connected via a user interface (18) with a touchscreen (19).wherein a document (20), a QR code (21), camera (23) and a biometric scanners (22) are connected to the control module (17).

3. Scanner device for verification of Covid 19 status, according to claim 1 and 2, characterized with that the communication module includes the following adapters: Wi-Fi, 3G/4G or Ethernet.

4. Scanner device for verification of Covid 19 status, according to claim 1, 2 and 3, **characterized in that** the biometric scanners (22) includes additional QR code reader for reading from smart devices.

5. Scanner device for verification of Covid 19 status, according to any of the preceding claims, **characterized in that** third parties in APIs of third parties (7) is a national Health Authority.
